# EUROPÄISCHE PATENTSCHRIFT

(11) **EP 2 251 063 B1**
(45) Veröffentlichungstag und Bekanntmachung des Hinweises auf die Patenterteilung: **21.11.2012**
(21) Anmeldenummer: 10156902.8
(22) Anmeldetag: 18.03.2010
(51) Int. Cl.: A61N 5/10, G21K 1/10

(54) **Verfahren zum Erstellen eines Therapieplans für eine Partikeltherapie sowie Filtervorrichtung für eine Partikeltherapieanlage**
Method for creating a treatment plan for particle therapy and filter device for a particle therapy assembly
Procédé d'élaboration d'un plan de traitement pour une thérapie à particules, ainsi que dispositif de filtre pour une installation de thérapie à particules

(30) Priorität: 13.05.2009 DE 102009021024
(43) Veröffentlichungstag der Anmeldung: 17.11.2010
(73) Patentinhaber: Siemens Aktiengesellschaft, 80333 München (DE)
(72) Erfinder: Gnutzmann, Tobias, 90459, Nürnberg (DE); Use, Tim, 90469, Nürnberg (DE)

(56) Entgegenhaltungen:
- WO-A2-2005/081842
- DE-A1- 10 227 808
- US-A- 6 108 403

## Beschreibung

Verfahren zum Erstellen eines Therapieplans für eine Partikeltherapie sowie Filtervorrichtung für eine Partikeltherapieanlage

Die Erfindung betrifft ein Verfahren zum Erstellen eines Therapieplans für eine Partikeltherapie. Die Erfindung betrifft weiterhin eine Filtervorrichtung für eine Partikeltherapieanlage.

Bei einer Partikeltherapie, insbesondere von Krebserkrankungen, wird in einem geeigneten Beschleuniger ein Partikelstrahl, beispielsweise aus Protonen oder Schwerionen, wie zum Beispiel Kohlenstoffionen, erzeugt. Der Partikelstrahl wird in einer Strahlführung in einen Behandlungsraum geführt und tritt dort über ein Austrittsfenster ein. In einer besonderen Ausführung kann der Partikelstrahl von einem Beschleuniger abwechselnd in verschiedene Behandlungsräume gelenkt werden. In dem Behandlungsraum ist ein zu therapierender Patient zum Beispiel auf einem Patiententisch positioniert und ggfs. mittels einer Immobilisierungseinrichtung immobilisiert.

Die Bestrahlung des Zielgebiets, gewöhnlich ein Tumor, erfolgt in der Regel schichtweise. In Abhängigkeit von seiner Energie reicht der Partikelstrahl unterschiedlich tief in das Gewebe, so dass das Gewebe sozusagen in scheibenförmige Abschnitte oder Schichten gleicher Eindringtiefe unterteilt werden kann. Der fokussierte Partikelstrahl wird dann über die einzelnen Schichten des Zielgebiets bewegt, was als "beam scanning" bezeichnet wird, so dass innerhalb einer Schicht mehrere Punkte bestrahlt werden, die zum Beispiel auf einem Rastergitter liegen. Indem die Strahlungsintensität bzw. die Energien richtig ausgewählt werden, können auch kompliziert geformte Bereiche genau bestrahlt werden. Die Anordnung der zu bestrahlenden Schichten und Punkte wird so gewählt, dass sich die geplante Dosisverteilung erzielen lässt.

Bei der Bestrahlung mit einem Beschleuniger ist eine minimale Energie erforderlich, die der Ring-Beschleuniger im Hinblick auf eine gute Strahlqualität zur Verfügung stellen soll. Diese minimale Energie entspricht einer Wasseräquivalent-Dicke von z.B. 20mm, d.h. der Stahl reicht 20mm ins Gewebe. Aufgrund der minimalen erforderlichen Energie können anhand der aktiven Energiemodulation des Beschleunigers nur Zielgebiete behandelt werden, die sich 20mm oder tiefer unter der Hautoberfläche befinden. Um das Gewebe zwischen der Haut und 20mm unter der Hautoberfläche zu erreichen, ist ein passiver Filter, ein so genannter "Rangeshifter" vorgesehen, der den Partikelstrahl bremst, so dass die Bestrahlung von hautnahen Tumoren ermöglicht ist. Ein solcher Filter ist in der Regel aus einem wasseräquivalenten Material wie, z.B. PMMA, nach Art einer Platte ausgebildet, ist zwischen den Strahlausgang und dem Patienten gehalten und weist eine Stärke auf, die eben diesen z.B. 20mm Wasseräquivalenz entspricht. Somit erfolgt eine Abbremsung der Strahlenergie innerhalb der Filterplatte und die Bestrahlung einer zu behandelnden Körperregion unmittelbar hinter der Filterplatte wird ermöglicht.

Da durch die Wechselwirklung zwischen dem Partikelstrahl und den Atomen der Filterplatte Streueffekte auftreten, wird angestrebt, die Filterplatte möglichst unmittelbar an den Patienten zu positionieren. Des Weiteren muss die Orthogonalität der Filterplatte zum Partikelstrahl gewährleistet sein.

Bei den heutigen Beschleunigern ist der Filter für gewöhnlich im Bereich des Austrittsfensters verstellbar angeordnet und beim Herausfahren des Filters für die Therapie ergibt sich häufig das Problem, dass die Filterplatte mit dem Patiententisch kollidiert.

Zur Behandlung eines Patienten mit dem Partikelstrahl wird zuvor ein Therapieplan erstellt. In diesem wird festgelegt welche Schichten des Zielgebietes (Tumors) mit welcher Dosis und aus welcher Richtung bestrahlt werden sollen. Das Ziel ist es dabei den Tumor möglichst effizient zu bestrahlen, wobei Risikoorgane wie beispielsweise ein Sehnerv oder Teile des Gehirns aus der Bestrahlung ausgenommen werden oder die applizierte Dosis im gesunden Gewebe des "Einstrahlungskanals" minimiert wird.

Bei heutigen Therapieplanungssystemen wird der "Rangeshifter", der als eine starre Platte mit einer konstanten wasseräquivalenten Dichte ausgebildet ist, im Therapieplan vorgesehen. Dabei werden die reale Dicke, Qualität und Homogenität des Plattenmaterials nicht vom Planungssystem gemessen, sondern vor dem ersten Einsatz der Filterplatte werden ihre Eigenschaften in einem aufwändigen Vorgang bestimmt und bei den nachfolgenden Therapien als konstante Größen herangezogen.

Die WO 2005/081842 A2 offenbart ein Strahlentherapiegerät, das mit einem MRI-System kombiniert ist und in welchem ein Patient immobilisiert werden kann. Als mögliche Strahlungsquellen sind u.a. Protonen, Schwerionen und Neutronen genannt.

Der Erfindung liegt die Aufgabe zugrunde eine Verbesserung der Therapieplanung für eine Bestrahlung mit einer Partikeltherapieanlage zu ermöglichen.

Die Aufgabe wird erfindungsgemäß gelöst durch ein Verfahren zum Erstellen eines Therapieplans für eine Partikeltherapie, bei dem ein Filter zum Einstellen der Eindringtiefe des Partikelstrahls sowie eine Immobilisierungseinrichtung zum Immobilisieren einer zu behandelnden Körperregion eines Patienten eingesetzt werden, wobei der Filter aus einer verformbaren Filtermasse ausgebildet, die unter Anpassung ihrer Geometrie an die Geometrie der Immobilisierungseinrichtung zumindest teilweise auf die Immobilisierungseinrichtung aufgebracht wird und wobei ein Planungsdatensatz mit zumindest einer Aufnahme des Filters gewonnen wird, so dass anhand der Aufnahme die Eigenschaften des Filters ermittelt und beim Therapieplan herangezogen werden.

Das Verfahren basiert auf der Anwendung einer Filtermasse bei einer Partikeltherapie, die auf die Immobilisierungseinrichtung aufgetragen wird und deren Eigenschaften bei der Therapieplanung berücksichtigt werden, so dass die Behandlung sehr präzise geplant wird. Die Filtermasse ist dabei leicht verformbar, weist eine relativ hohe Homogenität bezüglich ihrer Wasseräquivalenz auf und ist langzeitstabil bezüglich ihres Wassergehalts, d.h. das Material trocknet während der Behandlung nicht aus und schrumpft nicht.

Im Vorfeld der Behandlung wird der Patient für gewöhnlich mittels der Immobilisierungseinrichtung immobilisiert und ein Planungsscan der zu behandelnden Körperregion, insbesondere mittels eines Computertomographie(CT)-Geräts, wird gewonnen. Insbesondere werden die Aufnahmen mit dem CT-Gerät gewonnen, wenn die zu behandelnde Körperregion in einem Isozentrum der Partikeltherapieanlage positioniert ist und mittels der Immobilisierungseinrichtung in dieser Stellung festgehalten ist. Dabei werden Daten über die Form, Größe und/oder die Position des Tumors gewonnen und in einem Planungsdatensatz gespeichert. Gemäß dem vorgeschlagenen Verfahren wird außerdem im selben oder in einem getrennten Planungsscan die auf der Immobilisierungseinrichtung aufgetragene Filtermasse aufgenommen, so dass ebenfalls genaue Daten über den Filter vorliegen. Die Daten über den Filter werden anschließend bei der Therapieplanung verwendet, wobei rechnerisch ermittelt wird, von welcher Richtung, mit welcher Energie, wie lange durch den Filter bestrahlt wird.

Die Immobilisierungseinrichtung ist dabei ein Lagerungs- und Positionierungssystem beispielsweise aus Schaumstoff oder Karbon, welches heutzutage in der Strahlentherapie breite Anwendung findet, um an den verschiedenen Bestrahlungstagen die genaue Positionierung des Patienten bezüglich des Isozentrums der Partikeltherapieanlage während der Bestrahlung zu gewährleisten. Beispiel für eine solche Immobilisierungseinrichtung ist eine Immobilisierungsmaske zum Auflegen auf das Gesicht des Patienten, die insbesondere auf einer kleinen Kopfplatte oder auf einer Kopfschulterplatte befestigt wird, so dass eine Kopfbewegung des Patienten verhindert wird.
Die Erfindung geht von der Überlegung aus, dass durch den Einsatz eines Filters, der aus einer verformbaren Filtermasse ausgebildet ist, welche die starren Filterplatten ersetzt, die Filtermasse unproblematisch patientennah angebracht werden kann, indem sie direkt auf die Immobilisierungseinrichtung aufgebracht ist. Somit ist der Filter entkoppelt von dem Beschleuniger, es ist keine aufwendige Bewegungsmechanik zum Verfahren des Filters erforderlich, so dass sich Kollisionen mit dem Patiententisch oder der restlichen Mechanik des Beschleunigers vermeiden lassen. Die Wartung der Partikeltherapieanlage ist dadurch vereinfacht, da die Partikeltherapieanlage nicht mehr mit der Wartung des Filters oder einer Bewegungsmechanik des Filters gekoppelt ist. Insbesondere wird die Filtermasse auf die Immobilisierungseinrichtung vor der Behandlung außerhalb des Behandlungsraums aufgebracht, wodurch der Aufenthalt des Patienten im Behandlungsraum gekürzt ist und eine Optimierung des Behandlungsvorganges im Hinblick auf eine aufeinanderfolgende Bestrahlung mehrerer Patienten vorliegt.

Die Filtermasse wird außerdem in verschiedenen Dicken und Größen individuell auf die Patienten aufgebracht. Zumeist ist es ausreichend nur einen Teilbereich der Immobilisierungseinrichtung mit der Filtermasse zu bedecken, beispielsweise einen Teilbereich mit einem Durchmesser von ca. 15cm.

Da die Filtermasse sich an die Kontur der Immobilisierungseinrichtung und somit des Patienten anpasst, ist ein solcher Filter für Behandlungen von verschiedenen Richtungen uneingeschränkt geeignet.

Vorzugsweise wird der Therapieplan unter Berücksichtigung der Dickenverteilung und/oder der Homogenität der Filtermasse erstellt. Da im Planungsdatensatz genaue Informationen über die Dicke der Filtermasse an jeder Stelle enthalten ist und diese Informationen bei der Therapieplanung berücksichtigt werden, sind die Anforderungen an die Geometrie gering und kleine Abweichungen von einer gewünschten Dicke sind zulässig. Mittels des Planungsscans wird außerdem Information über die Homogenität der Filtermasse in den unterschiedlichen Bereichen des Filters erhalten, so dass die Anforderungen an die Homogenität bei der Herstellung des Filters ebenfalls reduziert sind.

Zweckdienlicherweise wird als Immobilisierungseinrichtung eine Immobilisierungsmaske angewendet. Die Immobilisierungsmaske ist aus einem thermoplastischen Maskenmaterial ausgebildet, welches bei einer Wassertemperatur von ca. 65° in einen verformbaren Zustand gebracht und dann der entsprechenden Patientenkontur angepasst wird. Es wird dabei insbesondere innerhalb eines Planungsschrittes die Immobilisierungsmaske an den Patienten angepasst und die Filtermasse darauf aufgetragen.

Als Filtermasse ist eine Masse geeignet, welche, nachdem sie die Form der Immobilisierungseinrichtung angenommen hat, erstarrt. Alternativ bleibt die Filtermasse nach ihrem Auftragen relativ weich, so dass sie unter Umständen z.B. unter dem Einfluss ihres eigenen Gewichts ihre Form oder Position verändert. In diesem Fall wird auf die Oberfläche der Filtermasse bevorzugt eine Abdeckung aufgetragen, welche dazu dient, die Filtermasse zu fixieren. Die Abdeckung weist eine im Vergleich zur Filtermasse viel geringere Stärke von ca. 1:20 auf, so dass ihr Einfluss auf die Energie des Partikelstrahls beim Durdringen des Abdeckungsmaterials minimal ist. Die Abdeckung kann beispielsweise aus Kunststoff oder aus dem gleichen Material wie die Immobilisierungseinrichtung sein und eine netzartige Struktur aufweisen oder nach Art einer Fixierschicht oder Fixierfolie ausgebildet sein.

Gemäß einer bevorzugten Variante wird ein erster Planungsdatensatz mit Aufnahmen der zu behandelnden Körperregion, welche mittels der Immobilisierungseinrichtung immobilisiert ist, gewonnen, ein zweiter Planungsdatensatz mit Aufnahmen der Immobilisierungseinrichtung mit dem Filter wird gewonnen und zur Optimierung des Therapieplans werden beide Planungsdatensätze miteinander abgeglichen. Beim zweiten Planungsscan werden dabei lediglich die Immobilisierungseinrichtung und der Filter erfasst, ohne dass diese auf den Patienten aufgelegt sind. Diese Vorgehensweise ist besonders effizient, wenn bereits Daten über den Tumor vorliegen, die lediglich durch die Informationen über den Filter ergänzt werden sollen, um die Behandlung optimal zu planen. Im ersten Schritt wird ein Planungsscan des Patienten mit der Immobilisierungseinrichtung durchgeführt, so dass die genaue Geometrie der Immobilisierungseinrichtung während der Bestrahlung erfasst wird. Erst nachdem bekannt ist an welcher Stelle der Partikelstrahl die Immobilisierungseinrichtung durchdringt, wird die Filtermasse auf die Immobilisierungseinrichtung aufgetragen und durch einen weiteren Planungsscan erfasst.

Es ist möglich, dass sich die Geometrie der Immobilisierungseinrichtung geringfügig verändert, wenn sich der Patient darin befindet - beispielsweise kann sich die Immobilisierungseinrichtung aufweiten. Um solche minimale Veränderungen bei der Therapieplanung zu berücksichtigen, wird gemäß einer alternativen Variante ein gemeinsamer Planungsdatensatz mit Aufnahmen der zu behandelnden Körperregion mit angebrachter Immobilisierungseinrichtung und dem Filter gewonnen und der Therapieplan wird auf Grundlage dieses Planungsdatensatzes erstellt. Ein gemeinsamer Datensatz, der durch einen einzigen Planungsscan des Patienten, der Immobilisierungseinrichtung und des Filters erhalten wird, ist auch sinnvoll, wenn z.B. aus einem Diagnosedatensatz bereits bekannt ist, dass bei der Bestrahlung ein Filter erforderlich ist.

Die Aufgabe wird weiterhin erfindungsgemäß gelöst durch eine Filtervorrichtung für eine Partikeltherapieanlage, umfassend einen Filter zum Einstellen der Eindringtiefe des Partikelstrahls und eine Immobilisierungseinrichtung zum Immobilisieren einer zu behandelnden Körperregion eines Patienten, wobei der Filter eine verformbare Filtermasse aufweist, die unter Anpassung ihrer Geometrie an die Geometrie der Immobilisierungseinrichtung auf der Immobilisierungseinrichtung auftragen ist.

Die in Bezug auf das Verfahren aufgeführten Vorteile und bevorzugten Ausgestaltungen sind auf die Filtervorrichtung zu übertragen.

Zweckdienlicherweise ist die Filtermasse eine Gelatinemasse. Die Gelatinemasse kann einfach hergestellt werden und ist lebensmittelecht, so dass sie unproblematisch patientennah aufgetragen werden kann. Die zum Ausbilden des Filters erforderliche Menge kann außerdem auf einfache Weise zugeschnitten werden.

Zum Fixieren der Filtermasse ist vorteilhafterweise auf der Oberfläche der Filtermasse eine Abdeckung, wie vorhergehend beschrieben, aufgetragen.

Ein Ausführungsbeispiel der Erfindung wird im Folgenden anhand einer Zeichnung näher erläutert. Hierin zeigen:
- FIG 1: eine stark vereinfachte schematische Darstellung einer Partikeltherapieanlage, und
- FIG 2: eine schematische Darstellung des Aufbaus einer Filtervorrichtung.

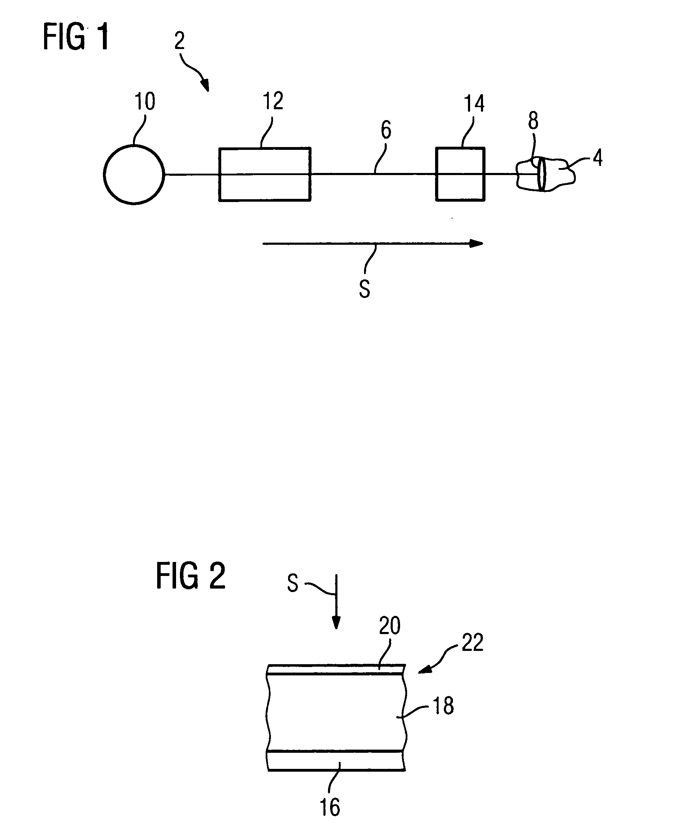

In FIG 1 ist eine Prinzipskizze einer Partikeltherapieanlage 2 gezeigt. Die Partikeltherapieanlage 2 wird eingesetzt, um eine zu behandelnde Körperregion 4, d.h. ein Tumorgewebe eines nicht näher gezeigten Patienten mit Hilfe eines Partikelstrahls 6 schrittweise zu bestrahlen. Dabei wird bei jedem Schritt ein scheibenförmiger Abschnitt 8 des Tumors 4, weiterhin auch Schicht genannt, behandelt.

Der Partikelstrahl 6 wird in einem Beschleuniger 10 generiert, der von einer Steuereinheit 12 angesteuert wird. Der Beschleuniger 10 liefert die Partikel mit einer Energie, die für die aktuell zu bestrahlende Schicht 8 erforderlich ist. Die Steuereinheit 12 umfasst u.a. eine hier nicht gezeigte Rasterscanvorrichtung, die den Strahl 6 sowohl in horizontaler als auch in vertikaler Richtung ablenkt, um das Tumorgewebe 4 innerhalb der Schicht 8 abzutasten. Dazu umfasst die Rasterscanvorrichtung beispielsweise zwei Magnetenpaare.

Wenn der Tumor 4 sich zumindest teilweise auf einer Tiefe <20mm unter der Hautoberfläche befindet, kann die Energie des Beschleunigers 10 nicht derart eingestellt werden, dass die Partikel auf einer geringeren Tiefe in den Patienten eindringen. In diesem Fall ist eine Filtervorrichtung 14 zum passiven Einstellen der Energie der Partikel erforderlich, welche zwischen einem Austrittsfenster der Partikeltherapieanlage 2 und dem Patienten positioniert ist. Eine solche Filtervorrichtung 22, die in unmittelbarer Nähe des Patienten und insbesondere auf dem Patienten angeordnet ist, ist in FIG 2 gezeigt. Die Filtervorrichtung 22 weist eine Immobilisierungseinrichtung 16 auf, die im direkten Kontakt mit dem Patienten steht. Wenn der Tumor 4 sich z.B. im Kopfbereich befindet, ist die Immobilisierungseinrichtung 16 eine Immobilisierungsmaske aus einem thermoplastischen Material, die an die Gesichtskonturen des Patienten angepasst ist.

Auf der Immobilisierungseinrichtung 16 ist eine Schicht aus einer verformbaren Filtermasse 18, hier eine Gelatinemasse, aufgetragen, so dass die Partikel durch Stöße mit den Gelatineatomen abgebremst werden. Durch die Gelatinemasse kann somit die Eindringtiefe des Partikelstrahls 6 verkleinert werden.

An der Oberfläche der Gelatinemasse 18 ist eine Abdeckung 20 nach Art einer Fixierschicht aus Kunststoff vorgesehen, welche die weiche, verformbare Gelatinemasse 18 festhält. Alternativ kann die Filtermasse 18 starr sein, so dass eine Abdeckung 20 nicht nötig ist. Die Filtermasse 18 und die Abdeckung 20 bilden hierbei einen Filter 22 zum Einstellen der Eindringtiefe des Partikelstrahls 6. Der Filter 22 kann die komplette Immobilisierungsmaske 16 bedecken, bevorzugt ist er jedoch nur auf einem Teilbereich der Immobilisierungsmaske 16 aufgetragen.

Die Filtervorrichtung 14 wird im Vorfeld der Behandlung bei einem Planungsscan z.B. mittels eines hier nicht näher gezeigten CT-Geräts erfasst, so dass die Dicke der Gelatineschicht 18 sowie ihre Homogenität ermittelt und bei der Therapieplanung berücksichtigt werden. Im Planungsscan können Aufnahmen des Tumors 4 gewonnen werden, auf welchen ebenfalls die Immobilisierungseinrichtung 16 und der Filter 22 miterfasst sind.

In der Regel ist es jedoch so, dass in einem ersten Planungsschritt die Filtervorrichtung 14 nicht auf den Patienten aufgelegt wird und durch einen ersten Planungsscan des immobilisierten Patienten ein Planungsdatensatz erhalten wird, der lediglich Informationen über den Tumor 4 und der Immobilisierungsmaske 16 enthält. Erst nach der Auswertung dieser ersten Aufnahmen wird ein zweiter Planungsdatensatz erhalten, der Aufnahmen der Filtervorrichtung 14, d.h. der Immobilisierungseinrichtung 16 und dem Filter 22, enthält. Bei der Auswertung des zweiten Planungsscans werden insbesondere die Dickenverteilung der Filtermasse 18, ihre Qualität und ihre lokale Homogenität bestimmt. Durch s.g. "Matching" werden beide Planungsdatensätze zusammengeführt und auf dieser Basis wird der Therapieplan erstellt.

Dadurch, dass die Eigenschaften des Filters 22 vor der Behandlung ermittelt wird, sind die Anforderungen an seine Geometrie und seine Homogenität nicht so hoch wie z.B. bei einer herkömmlichen Filterplatte aus PMMA, trotzdem ist die Einstellung der Tiefe, die der Partikelstrahl 6 im Patientenkörper erreicht, sehr präzis.

### Bezugszeichenliste

- 2: Partikeltherapieanlage
- 4: zu behandelnde Körperregion
- 6: Partikelstrahl
- 8: Abschnitt
- 10: Beschleuniger
- 12: Steuereinheit
- 14: Filtervorrichtung
- 16: Immobilisierungseinrichtung
- 18: Filtermasse
- 20: Abdeckung
- 22: Filter
- S: Strahlungsrichtung

## Patentansprüche

1. Verfahren zum Erstellen eines Therapieplans für eine Partikeltherapie, bei dem eine Immobilisierungseinrichtung (16) zum Immobilisieren einer zu behandelnden Körperregion (4) eines Patienten eingesetzt wird,
**dadurch gekennzeichnet, dass**
ein Filter (22) zum Einstellen der Eindringtiefe des Partikelstrahls (6) eingesetzt wird, wobei der Filter (22) aus einer verformbaren Filtermasse (18) ausgebildet ist, die unter Anpassung ihrer Geometrie an die Geometrie der Immobilisierungseinrichtung (16) zumindest teilweise auf die Immobilisierungseinrichtung (16) aufgebracht wird und wobei ein Planungsdatensatz mit zumindest einer Aufnahme des Filters (22) gewonnen wird, so dass anhand der Aufnahme die Eigenschaften des Filters (22) ermittelt und beim Therapieplan herangezogen werden.

2. Verfahren nach Anspruch 1,
bei dem der Therapieplan unter Berücksichtigung der Dickenverteilung und/oder der Homogenität der Filtermasse (18) erstellt wird.

3. Verfahren nach einem der vorhergehenden Ansprüche,
bei dem als Immobilisierungseinrichtung (16) eine Immobilisierungsmaske angewendet wird.

4. Verfahren nach einem der vorhergehenden Ansprüche,
bei dem auf die Oberfläche der Filtermasse (18) eine Abdeckung (20) aufgetragen wird.

5. Verfahren nach einem der Ansprüche 1 bis 4,
bei dem ein erster Planungsdatensatz mit Aufnahmen der zu behandelnden Körperregion (4), die mittels der Immobilisierungseinrichtung (16) immobilisiert ist, gewonnen wird, ein zweiter Planungsdatensatz mit Aufnahmen der Immobilisierungseinrichtung (16) mit dem Filter (22) gewonnen wird, und zur Optimierung des Therapieplans beide Planungsdatensätze miteinander abgeglichen werden.

6. Verfahren nach einem der Ansprüche 1 bis 4,
bei dem ein gemeinsamer Planungsdatensatz mit Aufnahmen der zu behandelnden Körperregion (4) mit angebrachter Immobilisierungseinrichtung (16) und dem Filter (22) gewonnen wird und der Therapieplan auf Grundlage dieses Planungsdatensatzes erstellt wird.

7. Filtervorrichtung (14) für eine Partikeltherapieanlage, umfassend eine Immobilisierungseinrichtung (16) zum Immobilisieren einer zu behandelnden Körperregion eines Patienten, **dadurch gekennzeichnet, dass**
die Filtervorrichtung einen Filter (22) zum Einstellen der Eindringtiefe des Partikelstrahls umfasst, wobei der Filter (22) eine verformbare Filtermasse (18) aufweist, die unter Anpassung ihrer Geometrie an die Geometrie der Immobilisierungseinrichtung (16) auf die Immobilisierungseinrichtung (16) auftragen ist.

8. Filtervorrichtung nach Anspruch 7,
wobei die Filtermasse (18) eine Gelatinemasse ist.

9. Filtervorrichtung nach Anspruch 7 oder 8,
wobei auf der Oberfläche der Filtermasse (18) eine Abdeckung (20) aufgetragen ist.

## Claims

1. Method for creating a therapy plan for a particle therapy, in which an immobilisation facility (16) is used to immobilise a body region (4) of a patient to be treated,
**characterised in that**
a filter (22) is used to set the penetration depth of the particle beam (6), wherein the filter (22) is embodied from a deformable filter mass (18), which, by adjusting its geometry to the geometry of the immobilisation facility (16), is applied at least partially to the immobilisation facility (16) and with a planning data record being obtained with at least one recording of the filter (22), so that with the aid of the recording, the properties of the filter (22) are determined and used for the therapy plan.

2. Method according to claim 1,
in which the therapy plan is created by taking account of the thickness distribution and/or the homogeneity of the filter mass (18).

3. Method according to one of the preceding claims, in which an immobilisation mask is used as an immobilisation facility (16).

4. Method according to one of the preceding claims,
in which a cover (20) is applied to the surface of the filter mass (18).

5. Method according to one of claims 1 to 4,
in which a first planning data record is obtained with recordings of the body region (4) to be treated, which is immobilised by means of the immobilisation facility (16), a second planning data record is obtained with recordings of the immobilisation facility (16) with the filter (22) and both planning data records are balanced with one another in order to optimise the therapy plan.

6. Method according to one of claims 1 to 4,
in which a shared planning data record with recordings of the body region (4) to be treated is obtained with the attached immobilisation facility (16) and the filter (22) and the therapy plan is created on the basis of this planning data record.

7. Filter apparatus (14) for a particle therapy installation, including an immobilisation facility (16) for immobilising a body region of a patient to be treated,
**characterised in that** the filter apparatus includes a filter (22) for setting the penetration depth of the particle beam, with the filter (22) comprising a deformable filter mass (18), which, by adjusting its geometry to the geometry of the immobilisation facility (16), is applied to the immobilisation facility (16).

8. Filter apparatus according to claim 7, with the filter mass (18) being a gelatin mass.

9. Filter apparatus according to claim 7 or 8, with a cover (20) being applied to the surface of the filter mass (18).

## Revendications

1. Procédé d'élaboration d'un plan de traitement pour une thérapie à particules, dans lequel on utilise un dispositif d'immobilisation (16) pour immobiliser une région du corps (4) d'un patient à traiter,
**caractérisé en ce que**
on utilise un filtre (22) pour régler la profondeur de pénétration du faisceau de particules (6), lequel filtre est formé d'une masse filtrante (18) déformable, qui est appliquée au moins partiellement sur le dispositif d'immobilisation (16) et dont la géométrie s'adapte à la géométrie du dispositif d'immobilisation (16), et on obtient un jeu de données de planification par au moins un cliché du filtre (22), permettant ainsi, à l'aide du cliché, de déterminer les caractéristiques du filtre (22) et d'en tenir compte dans le plan de traitement.

2. Procédé selon la revendication 1,
dans lequel on élabore le plan de traitement en considérant la distribution des épaisseurs et/ou l'homogénéité de la masse filtrante (18).

3. Procédé selon l'une des revendications précédentes,
dans lequel on utilise comme dispositif d'immobilisation (16) un masque d'immobilisation.

4. Procédé selon l'une des revendications précédentes,
dans lequel on applique un cache (20) sur la surface de la masse filtrante (18).

5. Procédé selon l'une des revendications 1 à 4,
dans lequel on obtient un premier jeu de données de planification par des clichés de la région du corps (4) à traiter, immobilisée au moyen du dispositif d'immobilisation (16), on obtient un deuxième jeu de données de planification par des clichés du dispositif d'immobilisation (16) muni du filtre (22), et on ajuste les deux jeux de données de planification pour optimiser le plan de traitement.

6. Procédé selon l'une des revendications 1 à 4,
dans lequel on obtient un jeu de données de planification commun par des clichés de la région du corps (4) à traiter munie du dispositif d'immobilisation (16) et du filtre (22), et on élabore le plan de traitement sur la base de ce jeu de données de planification.

7. Dispositif de filtre (14) pour une installation de thérapie à particules, comprenant un dispositif d'immobilisation (16) pour immobiliser une région du corps d'un patient à traiter,
**caractérisé en ce que**
le dispositif de filtre comprend un filtre (22) pour régler la profondeur de pénétration du faisceau de particules, lequel filtre (22) est constitué d'une masse filtrante (18) déformable, qui est appliquée sur le dispositif d'immobilisation (16) et dont la géométrie s'adapte à la géométrie du dispositif d'immobilisation (16).

8. Dispositif de filtre selon la revendication 7,
dans lequel la masse filtrante (18) est une masse de gélatine.

9. Dispositif de filtre selon la revendication 7 ou 8,
dans lequel un cache (20) est appliqué sur la surface de la masse filtrante (18).
